(19) Europäisches Patentamt
European Patent Office
Office européen des brevets

(11) EP 1 437 341 B1

(12) EUROPÄISCHE PATENTSCHRIFT

(45) Veröffentlichungstag und Bekanntmachung des Hinweises auf die Patenterteilung:
**23.02.2011 Patentblatt 2011/08**

(51) Int Cl.:
***C07C 209/10*** *(2006.01)* ***C07C 209/36*** *(2006.01)*
***C07C 211/55*** *(2006.01)* ***B01J 31/18*** *(2006.01)*

(21) Anmeldenummer: **03029846.7**

(22) Anmeldetag: **24.12.2003**

(54) **Verfahren zur Herstellung von Nitrodiphenylaminen**

Process for the preparation of nitrodiphenylamines

Procédé pour la préparation de diphénylamines

(84) Benannte Vertragsstaaten:
**AT BE BG CH CY CZ DE DK EE ES FI FR GB GR HU IE IT LI LU MC NL PT RO SE SI SK TR**

(30) Priorität: **07.01.2003 DE 10300125**

(43) Veröffentlichungstag der Anmeldung:
**14.07.2004 Patentblatt 2004/29**

(73) Patentinhaber: **LANXESS Deutschland GmbH**
**51369 Leverkusen (DE)**

(72) Erfinder:
- **Kunz, Klaus, Dr.**
  **40235 Düsseldorf (DE)**
- **Haider, Joachim, Dr.**
  **50674 Köln (DE)**
- **Ganzer, Dirk**
  **51373 Leverkusen (DE)**
- **Scholz, Ulrich, Dr.**
  **45475 Mülheim an der Ruhr (DE)**
- **Sicheneder, Adolf, Dr.**
  **25551 Hohenlockstedt (DE)**

(56) Entgegenhaltungen:
**WO-A-02/085838 WO-A-03/066570**
**US-A- 4 404 400 US-A1- 2001 012 906**
**US-B1- 6 180 788**

- **DATABASE WPI Section Ch, Week 198819 Derwent Publications Ltd., London, GB; Class A41, AN 1988-129550 XP002273823 & JP 63 072658 A (SUMITOMO CHEM IND KK) 2. April 1988 (1988-04-02)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft ein Verfahren zur Herstellung von Nitrodiphenylaminen durch Umsetzung von Nitrohalogenen mit Anilinen, einer Base und einem Katalysator sowie ein Verfahren zur Herstellung von Aminodiphenylamin durch Hydrierung des intermediär hergestellten Nitrodiphenylamins.

N-substituierte Aniline sind bedeutende Zwischenprodukte für die Herstellung von Agro- und Feinchemikalien.

Wie in Kirk-Othmer, Encyclopedia of Chemical Technology, 4th Edition, 1992, Vol 3, Seite 424-456 und in Ullmann's Encyclopedia of Industrial Chemistry, 5th Edition, Vol A3, 1985, Seite 91-111 beschrieben ist 4-Aminodiphenylamin (4-ADPA) ein wichtiges Vorprodukt zur Synthese von Alterungsschutzmitteln und Stabilisatoren in der Gummi- und Polymerindustrie.

Hartwig beschreibt in Angew. Chem., Int. Ed. 1998, 37, 2046-2067 und Buchwald in Top. Curr. Chem. 2002, 219,131-209, dass die Synthese von N-substituierten Aniline auch durch Übergangsmetall-katalysierte Kupplung von aktivierten Chlor-, Brom- oder Iodaromaten mit primären oder sekundären Aminen gegebenenfalls in Gegenwart eines Palladiumkatalysators, eines Phosphans und einer Base erfolgen. kann.

In WO-A2 01/66248 wird offenbart, dass alternativ anstatt der komplexen Pd-Phosphan-Komplexe auch *N*-heterocyclische Pd-Carbenkomplexe eingesetzt werden können.

Der Nachteil der im oben zitierten Stand der Technik beschriebenen Synthesen ist die Verwendung von Palladium, welches schlecht verfügbar, starken Preisschwankungen unterworfen und dessen Rückgewinnung aufwändig ist. Aufgrund ihrer schlechten Verfügbarkeit und ihrer hohen Toxizität ist die Verwendung der im Stand der Technik zitierten Schriften Phosphanliganden ebenfalls problematisch.

DE-A 3 246 151, DE-A 3 501 698, DE-A 185 663, US-A 4 670 595, US-A 4 187 249, US-A 4 683 332 und US-A 4 187 248 offenbaren die Darstellung von N-substituierten Anilinen aus p-Nitrochlorbenzol in Gegenwart eines Säureakzeptors oder eines Neutralisierungsmittels mit Hilfe von Kupfer-Katalysatoren.

In US-A 5 840 982 wird beschrieben, dass die erste Stufe zur Herstellung von N-substituierten Anilinen meist mit Kupfer-Katalysatoren, die zweite mit davon unterschiedlichen Metallkomponenten, z.B. Nickel, durchgeführt wird.

Venkatamaran et. al. offenbart in Tetrahedron Letters, 2001, 42, 4791-4793, dass die Herstellung von Triarylaminen aus Diarylaminen und Iodaromaten unter Zuhilfenahme von definierten Kupfer-Phosphan-Komplexen in hohen Ausbeuten möglich ist. Die selektive Herstellung der ökonomisch sehr bedeutenden Diarylamine wird in dieser Veröffentlichung nicht beschrieben.

Buchwald et. al beschreibt in Organic Letters, 2002, 4, 581-584 die allgemeine Umsetzung von Halogenbenzolen mit Anilinen. Die Verwendung des Katalysators nach Formel (I) wird nicht offenbart.

US-A-4 404 400 und JP-A-63072658 offenbaren die Herstellung von Nitrodiphenylaminen durch Reaktion von Anilin mit Nitrohalogenbenzolen in Gegenwart von Basen wobei in beiden Dokumenten keine Katalysatoren gemäß det Formel (I) eingesetzt werden.

Es bestand daher das Bedürfnis, Katalysatoren zu entwickeln, die sich in vorteilhafter Weise für ein Verfahren zur Herstellung von Nitrodiphenylaminen, ausgehend von Arylchloriden und Arylbromiden, eignen.

Da Arylchloride in der nucleophilen aromatischen Substitution üblicherweise deutlich reaktionsträger als Aryliodide sind, müssen für eine solche Reaktion deutlich drastischere Bedingungen, wie hohe Temperaturen, gewählt werden. Dies geht im Allgemeinen mit einer deutlich verringerten Selektivität der Reaktion einher.

Die Aufgabe der vorliegenden Erfindung ist es daher ein Verfahren bereit zustellen, dass es ermöglicht Nitrohalogenbenzole mit Anilinen in hohen Ausbeuten und mit hoher Selektivität zu Nitrodiphenylaminen umzusetzen.

Die Aufgabe wird gelöst durch ein Verfahren zur Herstellung von Nitrodiphenylaminen, wobei Nitrohalogenbenzole mit Anilinen, einer Base und einem Katalysator der Formel (I)

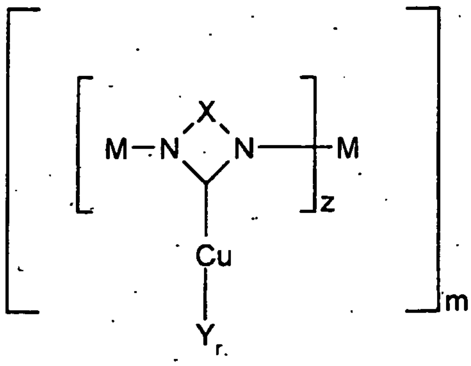

(I)

umgesetzt werden, wobei

X 1,2-Ethandiyl- oder 1,2-Ethendiyl-Rest ist und

M gleich oder verschieden sein kann und für $C_1$-$C_{19}$-Alkyl, $C_7$-$C_{19}$-Aralkyl, $C_6$-$C_{18}$Arylgruppen oder für $C_6$-$C_{19}$-Heteroaryle mit 1 bis 3 Stickstoffatomen steht, wobei zwei oder mehrere Reste M durch eine kovalente Brücke oder durch eine 1 bis 4 Kohlenstoffatome enthaltene Alkyliden-Brücke oder über einen Aryl- oder Heteroarylring beliebig verbrückt sein können,

Y für Halogen, Trifluoracetyl-, Trifluormethansulfonyl-, Nonafluorobutansulfonyl-, Cyanid-, Acetyl-, fluoriertes Acetylacetonyl-, Nitrat-, Arylsulfonyl-, Oxinat-, Phosphat-, Carbonat- oder Tetrafluorborat-Rest steht,

z für 1, 2 oder 3 steht,

m für ganze Zahlen von 1 bis 6 steht und

r 0, 1 oder 2 bedeutet.

Vorteilhaft wird die Base für das erfindungsgemäß Verfahren ausgewählt aus der Gruppe bestehend aus Hydrogencarbonate, Carbonate, Methanolate, Ethanolate, I-sopropylate, tert.-Butanolate, Phosphate, Fluoride, Silazane, Hydride und Acetate von Lithium, Natrium, Kalium und Cäsium.

Für das erfindungsgemäße Verfahren kann der Katalysator der Formel (I) auch in situ hergestellt werden.

Ein weiterer Gegenstand der Erfindung ist ein Verfahren zur Herstellung von Aminodiphenylamin, wobei das nach dem erfindungsgemäßen Verfahren hergestellte Nitrodiphenylamin ohne Isolierung hydriert wird

In der allgemeinen Formel (I) steht

X bevorzugt für eine 1,2-Ethendiylgruppe,

M bevorzugt für eine $C_1$-$C_{12}$ Alkylgruppe, $C_5$-$C_7$ Cycloalkylgruppe, $C_6$-$C_{12}$-Arylgruppe oder eine $C_5$-$C_{12}$ Heteroarylgruppe mit 1 bis 3 Stickstoffatomen im Ring oder für eine Methylgruppe, die durch eine kovalente Brücke oder durch eine 1 bis 4 Kohlenstoffatome enthaltene Alkyliden-Brücke oder über einen Aryl- oder Heteroarylring beliebig mit einem anderen Rest M verbrückt ist. Als $C_1$-$C_{12}$ Alkylgruppen sind sowohl verzweigte als auch unverzweigte Alkylgruppen zu verstehen. Bevorzugt sind Methyl-, Ethyl-, n-Propyl-, i-Propyl-, n-Butyl- und tert.-Butyl-. Als $C_5$-$C_6$ Cycloalkylgruppen sind bevorzugt Cyclopentyl- und Cyclohexylgruppen zu verstehen. Als $C_6$-$C_{12}$- Arylgruppen werden bevorzugt Phenyl, Biphenyl oder Naphthylrest eingesetzt. Bevorzugte $C_5$-$C_{12}$-Heteroarylrest mit 1 bis 3 Stickstoffatomen sind Pyridyl- oder Chinolinylreste.

Y bevorzugt für Chlor, Brom, Iod oder für einen Trifluormethylsulfonyl- oder einen Acetonyl-Rest.

z bevorzugt für 1 oder 2

m bevorzugt für 1, 2 oder 3

r bevorzugt für 1 oder 2

Für die Darstellung der Katalysatoren werden bevorzugt Kupferverbindungen mit einer Wertigkeitsstufe von 0, +I oder +II eingesetzt. Bevorzugte Ausgangsverbindungen für die Katalysatoren sind Kupferoxide, Kupferhalogenide, Kupfercyanide und Kupferacetate, Kupferactylacetonate in fluorierter oder nichtfluorierter Form, Kupfemitrate, Kupfertrifluormethansulfonate, Kupferarylsulfonate, Kupferoxinate, Kupferphosphate, besonders bevorzugt Kupfer(I)-chlorid, Kupfer(I)-bromid, Kupfer(I)-iodid, Kupfer(II)-bromid, Kupfer(II)-chlorid, Kupfer(II)-acetat, Kupfer(II)-oxid oder Kupfer (II)-acetylacetonat sowie Kupferpulver. Ganz besonders bevorzugt sind Kupfer(I)-chlorid, Kupfer(I)-bromid oder Kupfer (I)-trifluormethansulfonat.

Bevorzugt werden solche Katalysatoren, die zwei symmetrische, unsymmetrische oder zwei verbrückte Liganden enthalten. Besonders bevorzugt sind symmetrische Dialkylimidazolidene, Diarylimidazolidine, Diheteroarylimidazolidene, unsymmetrisch Arylalkylimidazoline sowie über Heteroaryle oder Alkyliden-Brücken verbrückte N-substituierte Imidazolidene. Ganz besonders bevorzugt sind N,N'-Dimethylimidazoliden, N,N'-Dicyclohexylimidazoliden, N,N'-Diphenylimidazoliden, N,N'-Di(2,6-diisopropyl)phenylimidazoliden, N,N'-Di(2,6-dimethyl)phenylimidazoliden, N,N'-Di(2,4,6-trimethyl)phenylimidazoliden, N,N'-Di(2-Pyridyl)imidazoliden, N-Benzyl-N'-methylimidazoliden und Liganden, die durch zweifache Deprotonierung von 1,3-Bis-[N-(N'-methyl)imidazoliummethyl]-5-methylbenzoldihalogenid, - 2,6-Bis-[N-(N'-methyl)-imidazoliummethyl]pyridindihalogenid, 1,2-Bis-[N-(N'-methyl)-imidazolium]-1,2-diphenylethanhalogenid oder Bis-[N-(N'-methyl)imidazolium]methandihalogenid gebildet werden.

Bevorzugte Katalysatoren der Formel (I) sind (N,N'-Dimethylimidazoliden)kupfer(II)bromid, (N,N'-Dicyclohexylimidazoliden)-kupfer[II]bromid, [N,N'-Di(2,4,6-trimethyl)phenylimidazoliden]kupfer(II)bromid, [N,N'-Di(2-Pyridyl)imidazoliden]-kupfer[II]bromid, (N-Benzyl-N'-methylimidazoliden)kupfer[II]bromid, { 1,3-Bis-[N-(N'-methyl)imidazolidenmethyl]-5-methylbenzol}kupfer[II]bromid, {1,3-Bis-[N-(N'-methyl)imidazoliden-methyl]-5-methylbenzol}kupfer[I]trilfluormethylsulfonat, {2,6-Bis-[N-(N'-methyl)-imidazolidenmethyl]pyridin}kupfer[II]bromid, {1,2-Bis-. [N-(N'-methyl)imid-azoliden]-1,2-diphenyleihan}kupfer[II]bromid, {Bis-[N-(N'-methyl)imidazoliden]-methan}kupfer[II]bromid. Besonders bevorzugt sind (N,N'-Dimethylimidazoliden)kupfer(II)bromid und {1,3-[Bis-(N-(N'-methyl)imidazolidenmethyl]-5-methylbenzol} kupfer[II]bromid.

Die genannten Katalysatoren können sowohl einzeln als auch im beliebigen Gemisch untereinander eingesetzt werden. Die günstigste Gemischzusammensetzung kann durch entsprechende Vorversuche bestimmt werden.

Die erfindungsgemäß einzusetzenden Katalysatoren der Formel (I) werden durch Deprotonierung von Liganden der Formel (II)

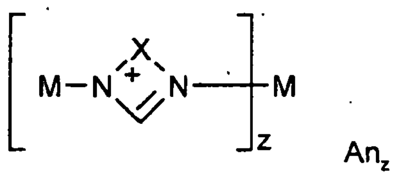

(II)

mit einer Base und anschließender Umsetzung mit einer Kupfer-Verbindungen der Formel (III)

$$Cu\text{-}Y_r \qquad (III)$$

wobei M, X, Y, z und r die in der Formel (I) angegebene Bedeutung besitzen, hergestellt.

Formel (II) steht dabei stellvertretend für die möglichen tautomeren Verbindungen, die vom Umfang der Erfindung ebenfalls umfasst sind.

Bevorzugt ist "An" ein Anion einer Säure, die einen pKa-Wert von 3 oder kleiner als 3 besitzt. Besonders bevorzugt ist "An" Hydrogensulfat, Chlorid, Bromid, Iodid, Tetrafluoroborat, Hexafluorophosphat oder ein halbes Äquivalent Sulfat.

Ganz besonders bevorzugt ist "An" für Chlorid, Bromid oder Iodid.

Als Basen für die Deprotonierung des Liganden werden Alkali- und/oder Erdalkalimetallalkoholate, -hydride und/oder -hydroxide eingesetzt. Bevorzugte Basen für die Deprotonierung der Liganden sind Natriummethanolat, Kaliumtertiär-butylat, Kaliumamylat, Natriumhydrid, Kaliumhydrid, Kaliumhydroxid, Natriumhydroxid und Bariumhydroxid. Besonders bevorzugt erfolgt dabei die Deprotonierung der Liganden durch Kalium-tert-Butylat.

Die Lösemittel für die Deprotonierung der Ligand sind inerte organische Lösemittel. Bevorzugt sind Ether, wie Diethylether, THF, aber auch Toluol, Xylol, Chloroform, Dichlormethan, Methanol und/oder Ethanol, wobei die Deprotonierung im Temperaturbereich von -50 bis 50°C durchgeführt wird. Besonders bevorzugte wird die Deprotonierung in Ethern bei Temperaturen zwischen -35° und Raumtemperatur durchgeführt.

Zur Herstellung der Katalysatoren der Formel (I) werden die Einsatzprodukte der Formel (II) und der Formel (III) in einem solchen molaren Verhältnis eingesetzt, dass sich der gewünschte Zielkatalysator der allgemeinen Formel (I) ergibt. Bevorzugt wird das molare Verhältnis der Imidazoliumsalze der Formel (II) zu den Kupfer-Verbindungen der Formel (III) im Bereich von 40:1 bis 0,5:1, besonders bevorzugt im Bereich von 5:1 bis 1:1, ganz besonders bevorzugt im Bereich von 4:1 bis 1:1 gewählt.

Die zur Herstellung der Katalysatoren benötigten Lösemittel sind die gleichen, die zur Deprotonierung des Liganden verwendet werden. Die günstigste Menge an einzusetzendem Lösemittel kann durch entsprechende Vorversuche bestimmt werden. Die Reaktionstemperatur wird nach vollständiger Deprotonierung auf eine Temperatur im Bereich von 10 bis 30°C erhöht. Bevorzugt wird die Temperatur innerhalb von 60 bis 180 min auf Temperaturen im Bereich von 15 bis 25°C erhöht.

Die Katalysatoren können sowohl als isolierte Verbindung dem erfindungsgemäßen Verfahren zur Herstellung von Diarylaminen zugesetzt werden, als auch in-situ während der Umsetzung der substituierten Benzole mit den Arylaminen umgesetzt werden.

Für das erfindungsgemäße Verfahren werden die Katalysatoren in Mengen von 0,02 Mol-% bis 10 Mol-%, bevorzugt 0,1 Mol-% bis 3 Mol-%, bezogen auf die Menge an eingesetzten Nitrohalogenbenzolen.

Als Nitrohalogenbenzole sind alle Nitrohalogenbenzole zu verstehen, die wenigstens ein Halogen ausgewählt aus der Gruppe bestehend aus Fluor, Chlor und Brom enthalten. Bevorzugte Halogene sind Fluor und Chlor. Die Nitrohalogenbenzole können neben der Halogen- und der Nitrogruppe noch ein oder mehrere Substituenten ausgewählt aus der Gruppe bestehend aus $C_1$-$C_{12}$-Alkylgruppen enthalten. Besonders bevorzugt sind die unsubstituierten Nitrohalogenbenzole und Nitroalkylhalogenbenzole mit unverzweigten Alkylgruppen. Ganz besonders bevorzugt sind die Nitrohalogenbenzole und Nitroalkylhalogenbenzole bei denen die Nitrogruppe in para, ortho oder metha-Stellung zum Halogen, insbesondere bevorzugt in para-Stellung, steht. Insbesondere ganz besonders bevorzugt sind 4-Nitro-2-methylchlorbenzol, 4-Nitro-3-methylfluorbenzol, 4-Nitrochlorbenzol, 3-Nitrochlorbenzol und 2-Nitrochlorbenzol. Hiervon ist insbesondere ganz besonders bevorzugt 4-Nitrochlorbenzol.

In das erfindungsgemäße Verfahren können neben Anilin auch alle dem Fachmann bekannte o-, m- und p-substituierten Aniline eingesetzt werden. Als Substituenten sind bevorzugt verzweigte oder unverzweigte $C_1$-$C_{29}$-Alkyl- , $C_2$-$C_{29}$-Alkenyl-, $C_1$-$C_{29}$-Acyl-, $C_1$-$C_{29}$-Alkylthio-, $C_1$-$C_{29}$-Alkyl-amino-, $C_1$-$C_{29}$-Alkoxyreste, $C_1$-$C_{29}$ Carbonsäureester mit 1 bis 29 C-Atomen im Carbonsäureteil und 1 bis 29 C-Atomen im Esterteil sowie Sulfonsäurereste mit 1 bis 9 Kohlenstoffatomen im Esterteil. Bevorzugt sind verzweigte oder unverzweigte Alkyl-, Alkenyl- oder Alkylthiogruppen mit den genannten Zahlen an Kohlenstoffatomen wie Methyl-, n-Butyl-, tert.-Butyl-, Octyl-, Decyl-, Dodecyl-, Myristyl-, Stearylgruppe. Besonders bevorzugte substituierte Aniline sind Vinylanilin, 4-tert. Butylanilin, p-Anisidin, o-Anisidin, o-Toluidin, p-Toluidin, Anthranilsäuremethylester, o-Aminobenzonitril, p-Aminobenzonitril und 4-Ethylanilin. Insbesondere ganz besonders bevorzugt wird Anilin eingesetzt.

Gemäß dem erfindungsgemäßen Verfahren werden im Allgemeinen pro Mol Nitrohalogenbenzol 1 bis 10 Mol, bevorzugt 1,5 bis 8 Mol, besonders bevorzugt 2 bis 6 Mol, des entsprechenden Anilins eingesetzt.

Als Basen werden im erfindungsgemäßen Verfahren Alkali- und/oder Erdalkalimetallcarbonate, -alkoholate, -phosphate, -fluoride und/oder -hydroxide eingesetzt, wobei insbesondere Kaliumcarbonat, Natriumcarbonat, Cäsiumcarbonat, Cäsiumhydrogencarbonat, Natriummethanolat, Kalium-tert.-butylat, Kaliumamylat, Cäsiumfluorid, Kaliumphosphat und Bariumhydroxid bevorzugt sind. Besonders bevorzugt werden Kaliumcarbonat, Natriumcarbonat, Cäsiumcarbonat und/oder Cäsiumhydrogencarbonat. Ganz besonders bevorzugt wird Kaliumcarbonat.

Die Basen können sowohl in unterstöchiömetrischer, stöchiometrischer Menge als auch im Überschuss bis zur zehnfachen äquivalenten Menge bezüglich des Nitrohalogenbenzols eingesetzt werden. Besonders bevorzugt werden die Basen in der 0,3 bis 2 äquivalenten Menge, bezogen auf das Nitrohalogenbenzol, eingesetzt.

Für das erfindungsgemäße Verfahren ist es von Vorteil, wenn die eingesetzten Basen durch Mahlung und/oder Trocknung vorbehandelt werden.

Die Mahlung kann im erfindungsgemäßen Verfahren in handelsüblichen Mühlen erfolgen. Das Mahlen bewirkt hierbei eine drastische Vergrößerung der spezifischen Oberfläche, die zu einer deutlichen Steigerung des Umsatzes führt. In vielen Fällen ist durch die Mahlung eine Vergrößerung der spezifischen Oberfläche um den Faktor 10 bis 20 zu beobachten.

Nach der Mahlung liegen die spezifischen Oberflächen der Basen bei ca. 0,1 bis 10 $m^2/g$, bevorzugt 0,2 bis 1 $m^2/g$ (BET).

Wegen der ausgeprägten hygroskopischen Eigenschaften der im erfindungsgemäßen Verfahren eingesetzten Basen, neigen vor allem die Phosphate und Carbonate zur mehr öder minder starken Aufnahme atmosphärischer

Bestandteile, wie Wasser und Kohlendioxid. Ab einer Aufnahme von ca. 30 Gewichtsprozent an atmosphärischen Bestandteilen ist ein deutlicher Einfluss auf die zu erreichenden Umsätze feststellbar. Daher ist neben der Mahlung häufig auch eine Trocknung der Basen notwendig.

Die Trocknung der Basen erfolgt dabei je nach Natur der verwendeten Base derart, dass unter vermindertem Druck von ca. 0,01 bis 100 mbar für mehrere Stunden auf Temperaturen von ca. 50 bis 200°C, bevorzugt 100 bis 160°C, erhitzt wird.

Bevorzugt wird das erfindungsgemäße Verfahren so durchgeführt, dass erst die Base bei Temperaturen im Bereich von 20 bis 250°C, bevorzugt bei Temperaturen von 110 bis 210°C getrocknet wird, die Temperatur im Reaktionsgefäß abgekühlt wird und anschließend die Nitrohalogenbenzole, Aniline und der Katalysator oder für den Fall, dass der Katalysator in-situ hergestellt wird, der Ligand nach Formel II mit der Kupferverbindung nach Formel III, hinzugefügt werden.

Das erfindungsgemäße Verfahren kann sowohl in Anwesenheit als auch in Abwesenheit eines zusätzlichen geeigneten Lösemittels durchgeführt werden. Als zusätzliches Lösemittel sind inerte, organische Kohlenwasserstoffe, wie Xylol und Toluol, bevorzugt. Weiterhin können die eingesetzten aromatischen Amine selbst als Lösemittel fungieren.

Die Menge der eingesetzten Lösemittel kann leicht durch entsprechende Vorversuche bestimmt werden.

Um die Ausbeute an Nitrodiphenylaminen zu steigern, kann wie in DE-A 2 633 811 und DE-A 3 246 151 beschrieben, das entstehende Reaktionswasser durch Zusatz eines geeigneten Schleppmittels wie Benzol, Toluol oder Xylol durch Destillation entfernt werden.

Die Durchführung des erfindungsgemäßen Verfahrens kann nach den dem Fachmann bekannten Methoden in kontinuierlicher oder diskontinuierlicher Weise erfolgen.

Die nach dem erfindungsgemäßen Verfahren hergestellten Nitrodiphenylamine können durch eine Hydrierung zu den entsprechenden Aminodiphenylaminen reduziert werden. Die Hydrierung erfolgt in der dem Fachmann bekannten Weise mit einem Reduktionsmittels, wie Wasserstoff, wobei das bereits vorhandene Kupfer aus Formel (I) nicht entfernt werden muss und gegebenenfalls ein geeigneter, inerten Katalysatorträgers hin.

Selbstverständlich ist es auch möglich, die Hydrierung in Gegenwart zusätzlicher Hydrierkatalysatoren, wie solche auf Nickel-, Palladium- oder Platin-Basis, durchzuführen. Für diese Katalysatoren können auch geeigneten Katalysatorträgers verwendet werden.

Geeignete Materialien für die Verwendung als Katalysatorträger sind alle dem Fachmann bekannten Katalysatorträger auf der Basis von Kohlenstoff, Elementoxiden, Elementcarbiden oder Elementsalzen in verschiedenen Anwendungsformen. Beispiele für kohlenstoffhaltige Träger sind Koks, Graphit, Ruß oder Aktivkohlen. Beispiele für die Elementoxid-Katalysatorträger sind $SiO_2$ - in Form von natürlicher oder synthetischer Kieselsäure, natürlichem oder synthetischem Quarz - $Al_2O_3$, bevorzugt $\alpha$- und $\gamma$-$Al_2O_3$, Tonerden, bevorzugt natürliche oder synthetische Alumosilicate wie Zeolithe, Schichtsilikate wie Bentonit, Montmorillonit, $TiO_2$, bevorzugt im Rutil oder Anatas-Typ, $ZrO_2$ MgO und ZnO. Beispiele für Elementcarbide und -salze sind SiC, $AlPO_4$, $BaSO_4$, $CaCO_3$. Grundsätzlich können sowohl synthetische Materialien als auch Träger aus natürlichen Quellen, wie Bimsstein, Kaolin, Bleicherden, Bauxite, Bentonite, Kieselgur, Asbest oder Zeolithe, verwendet werden. Bevorzugt werden Aktivkohlen und Si-, Al-, Mg-, Zr- und Ti-haltige Materialien als Trägermaterialien eingesetzt. Besonders bevorzugt ist Aktivkohle.

Selbstverständlich kann die Hydrierung auch mit anderen Reduktionsmethoden, wie sie dem Fachmann bekannt sind und in Reductions in Organic Chemistry, Second Edition, ACS Monograph 188, beschrieben werden, durchgeführt werden.

Für die Hydrierung kann das Nitrodiphenylamin entweder isoliert werden oder aber das aus dem erfindungsgemäßen Verfahren erhaltene Nitrodiphenylamin wird unter Entfernung von Alkalihalogeniden aber ohne weitere Aufarbeitung direkt hydriert.

Die Temperaturen bei der Hydrierung betragen ca. 0 bis 200°C, bevorzugt 40 bis 150°C. Die Wasserstoffdrücke für die Hydrierung liegen bei 0,1 bis 150 bar, bevorzugt bei 0,5 bis 70 bar, besonders bevorzugt bei 1 bis 50 bar.

Nach dem erfindungsgemäßen Verfahren werden die entsprechenden Aminodiphenylamine mit hoher Selektivität (> 95%) und Ausbeuten bis zu 97 % d. Th. erhalten.

## Beispiele

### Beispiel 1

Herstellung von {1,3-Bis-[N-(N'-methyl)imidazoliden-methyl]-5-methylbenzol}-kupfer[II]-bromid, $C_{17}H_{20}Br_2CuN_4$(x 2KBr)

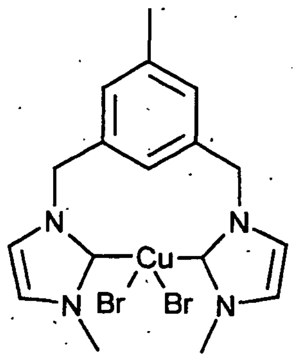

Unter Argonatmosphäre wird das Bisimidazoliumsalz (135mg, 0,31 mmol) in 10 ml Toluol gelöst und bei 0°C mit Kalium-tert.-butylat (71 mg, 0,63 mmol) versetzt. Nach 2 h wird Kupfer-II-bromid (70 mg, 0,31 mmol) zugesetzt und weitere 12 h gerührt. Anschließend wird das Lösungsmittel im Vakuum entfernt, das Produkt fällt als helles Pulver an. FD/MS: 343 (M-2Br, Hauptkomponente), 423 (M-Br), 503 (M+2H)

## Beispiel 2

Herstellung von {1,3-Bis-[N-(N'-methyl)imidazoliden-methyl]-benzol}kupfer[II]-bromid, $C_{15}H_{17}Br_2CuN_5$ (x 2KBr)

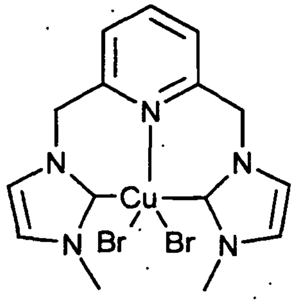

Unter Argonatmosphäre wird das Bisimidazoliumsalz (131mg, 0,31 mmol) in 10 ml Toluol gelöst und bei 0°C mit Kalium-tert.-butylat (71 mg, 0,63 mmol) versetzt. Nach 2 h wird Kupfer-II-bromid (70 mg, 0,31 mmol) zugesetzt und weitere 12 h gerührt. Anschließend wird das Lösungsmittel im Vakuum entfernt, das Produkt fällt als helles Pulver an. FD/MS: 330 (M-2Br, Hauptkomponente), 410 (M-Br), 490 (M+2H)

## Beispiel 3

Herstellung von [N,N'-Di(2-Pyridyl)imidazoliden]kupfer[II]bromid, $C_{13}H_{10}Br_2CuN_4$ (x KBr)

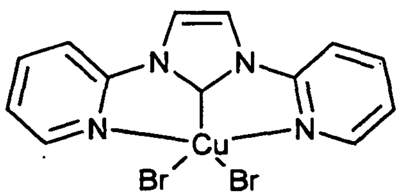

Unter Argonatmosphäre wird das Imidazoliumsalz (92mg, 0,31 mmol) in 10 ml Toluol gelöst und bei 0°C mit Kalium-tert.-butylat (36 mg, 0,31 mmol) versetzt. Nach 2 h wird Kupfer-II-bromid (70 mg, 0,31 mmol) zugesetzt und weitere 12 h gerührt. Anschließend wird das Lösungsmittel im Vakuum entfernt, das Produkt fällt als helles Pulver an.

FD/MS: 364 (M-Br, Hauptkomponente).

**Beispiel 4**

Herstellung von (N-Benzyl-N'-methylimidazoliden)kupfer[II]bromid,$C_{22}H_{24}Br_2$-$CuN_4$ (x 2KBr)

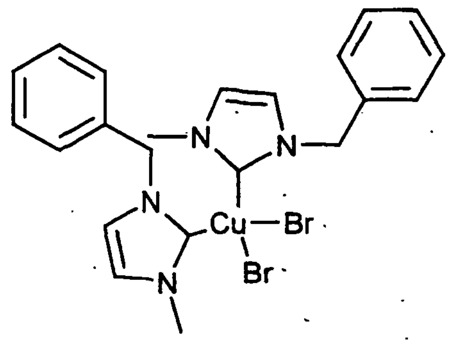

Unter Argonatmosphäre wird das Imidazoliumsalz (300mg, 1,2 mmol) in 10 ml Toluol gelöst und bei 0°C mit Kalium-tert-butylat (138 mg, 1,2 mmol) versetzt. Nach 2 h wird Kupfer-II-bromid (120 mg, 0,6 mmol) zugesetzt und weitere 12 h gerührt. Anschließend wird das Lösungsmittel im Vakuum entfernt, das Produkt fällt als helles Pulver an. FD/MS: 316 (M-2Br, Hauptkomponente).

**Beispiel 5**

Cu-Carben-katalysierte Herstellung von 4-Nitrodiphenylamin

In einem Mehrhalsrundkolben mit KPG-Rührer mit Teflonblatt, Vigreux-Kolonne, 55 ml-Wasserabscheider mit Xylol befüllt, sowie Stickstoffüberlagerung, Septum, Heizpilz und Isolierung wurden 288,9 g (3090 mmol) Anilin, 83,6 g (605 mmol) Pottasche (Vorbereitung: 60 sek. auf Stufe 1 und 3 x 60 sek. auf Stufe 2 des Labormixers gemahlen, wobei nach jeweils 60 sek. das Mahlgut durch Schütteln aufgelockert wurde), sowie 157,6 g (1000 mmol) 4-Chlornitrobenzol und 9,42 g (16,2 mmol) {1,3-Bis-[N-(N'-methyl)imidazoliden-methyl]-5-methylbenzol}kupfer[II]-bromid unter Rühren vorgelegt. Es entstand eine rot-braune Suspension, die bis auf Rückflusstemperatur erhitzt wurde (Rührer ca. 400 U/min, ganz leichter $N_2$-Strom). Daraufhin kam es zunächst nur zu einer geringen Wasserentwicklung, die sich im Laufe der Reaktion aber steigerte und dann auf niedrigem Niveau konstant blieb (insg. ca. 6,9 ml). Die Probenahme (unfiltriert) erfolgte alle 30 Minuten, die Proben wurden mittels HPLC (6-Punkt Kalibrierung) analysiert. Nach 390 Minuten wurde der Versuch beendet. Die Siedetemperatur des Gemisches lag während der gesamten Versuchsdauer bei etwa 192-199 °C. Dabei ergab sich ein Rest p-NCB-Wert von 6,0 Gew.-% (entspricht 84 % Umsatz), ein 4-NDPA-Gehalt von 32,3 Gew.-%, 4,4'-Dinitrotriphenylamin-Gehalt von 1,2 % und ein 4-NDPA-/Triarylamin-Verhältnis von 27 (entspricht 81 % 4-NDPA-Ausbeute und 96 % Selektivität bezogen auf p-NCB).

**Ergebnis:**

| Probe | Probe [Min] | p-NCB [Gew.-%] | 4-NDPA [Gew.-%] | Dreikern [Gew.-%] | Selektivität (NDPA/Dreikern) |
|---|---|---|---|---|---|
| 1 | 30 | 23,5 | 8,7 | 0,00 | |
| 2 | 62 | 21,0 | 13,2 | 0,19 | 71,0 |
| 3 | 90 | 18,7 | 15,3 | 0,14 | 112,8 |
| 4 | 121 | 17,0 | 18,4 | 0,36 | 50,7 |
| 5 | 150 | 15,8 | 21,0 | 0,43 | 48,5 |

(fortgesetzt)

| Probe | Probe [Min] | p-NCB [Gew.-%] | 4-NDPA [Gew.-%] | Dreikern [Gew.-%] | Selektivität (NDPA/Dreikern) |
|---|---|---|---|---|---|
| 6 | 180 | 15,1 | 24,6 | 0,55 | 44,5 |
| 7 | 210 | 12,4 | 25,1 | 0,37 | 67,4 |
| 8 | 240 | 9,8 | 23,7 | 0,66 | 36,0 |
| 9 | 270 | 8,5 | 25,5 | 0,76 | 33,6 |
| 10 | 302 | 8,2 | 30,0 | 1,00 | 30,1 |
| 11 | 347 | 6,6 | 32,3 | 1,09 | 29,6 |
| 12 | 362 | 6,0 | 32,3 | 1,20 | 26,9 |

**Beispiel 6**

Cu-Carben-katalysierte Herstellung von 4-Nitrodipheriylamin in Gegenwart von Cäsium

In einem Mehrhalsrundkolben mit KPG-Rührer mit Teflonblatt, Vigreux-Kolonne, 55 ml-Wasserabscheider mit Xylol befüllt, sowie Stickstoffüberlagerung, Septum, Heizpilz und Isolierung wurden 288,9 g (3090 mmol) Anilin, 83.6 g (605 mmol) Pottasche (Vorbereitung: 60 sek. auf Stufe 1 und 3 x 60 sek. auf Stufe 2 des Labormixers gemahlen, wobei nach jeweils 60 sek. das Mahlgut durch Schütteln aufgelockert wurde), sowie 157,6 g (1000 mmol) 4-Chlornitrobenzol und 0,50g (3,78 mmol) Cs als wässrige Lösung wurden unter Rühren vorgelegt. Es entstand eine rot-braune Suspension die bis auf Rückflusstemperatur erhitzt wurde (Rührer ca. 400 U/min, ganz leichter $N_2$-Strom). Nachdem das Wasser ausgekreist war, kühlte man auf ca. 80°C herunter und gab 4,71 g (8,1 mmol) {1,3-Bis-[N-(N'-methyl)-imidazoliden-methyl]-5-methylbenzol}kupfer[II]-bromid hinzu. Nachdem man wieder bis zum Rückfluss aufgeheizt hatte, kam es zunächst nur zu einer geringen Wasserentwicklung, die sich im Laufe der Reaktion aber steigerte und dann auf niedrigem Niveau konstant blieb (insg. ca. 7,2 ml). Die Probeentnahme (unfiltriert) erfolgte alle 30 Minuten, die Proben wurden mittels HPLC (6-Punkt Kalibrierung) analysiert. Nach 360 Minuten wurde der Versuch beendet. Die Siedetemperatur des Gemisches lag während der gesamten Versuchsdauer bei etwa 190-198°C. Dabei ergab sich ein Rest p-NCB-Wert von 6,2 Gew.-% (entspricht 84 % Umsatz), ein 4-NDPA-Gehalt von 33,9 Gew.-%, 4,4'-Dinitrotriphenylamin-Gehalt von 1,92 % und ein 4-NDPA-/Triarylamin-Verhältnis von 17,7 (entspricht 79 % 4-NDPA-Ausbeute und 95 % Selektivität bezogen auf p-NCB).

**Ergebnis:**

| Probe | Probe [Min] | p-NCB [Gew:-%] | 4-NDPA [Gew.-%] | Dreikern [Gew.-%] | Selektivität (NDPA/Dreikern) |
|---|---|---|---|---|---|
| 1 | 30 | 24,2 | 8,6 | 0,13 | 67,7 |
| 2 | 60 | 19,1 | 12,8 | 0,14 | 92,9 |
| 3 | 90 | 18,5 | 17,0 | 0,34 | 50,4 |
| 4 | 135 | 16,4 | 20,7 | 0,53 | 39,0 |
| 5 | 150 | 15,4 | 21,9 | 0,63 | 34,7 |
| 6 | 180 | 13,6 | 24,7 | 0,78 | 31,8 |
| 7 | 210 | 12,5 | 24,8 | 0,95 | 26,2 |
| 8 | 270 | 9,2 | 29,0 | 1,33 | 21,9 |
| 9 | 300 | 8,3 | 31,7 | 1,56 | 20,3 |
| 10 | 343 | 7,2 | 33,9 | 1,80 | 18,8 |
| 11 | 362 | 6,2 | 33,9 | 1,92 | 17,7 |

**Beispiel 7**

Herstellung von 4-Aminodiphenylamin

Zur Reaktionsmischung aus Beispiel 5 wurden nach Abkühlen auf 105°C 250 ml Wasser zugegeben, 15 min bei 80°C gerührt und in einen Scheidetrichter überführt. Nach erfolgter Phasentrennung wurden die abgetrennte organische Phase (500ml) mit 8ml KOH, 25 ml Salzwasser (aus wässriger Phase der Kondensation) und 4,5 g Raney-Nickel versetzt und in einen Hydrierautoklav überführt und bei einem Druck von 10 bar Wasserstoff in 400 min hydriert, wobei die Temperatur von 140°C erreicht wurde. Nach gaschromatographischer Untersuchung erhält man 91 % 4-Aminodiphenylamin (bezogen auf das eingesetzte 4-Nitrodiphenylamin).

**Patentansprüche**

1. Verfahren zur Herstellung von Nitrodiphenylaminen, wobei Nitrohalogenbenzole mit Anilinen, einer Base und einem Katalysator der Formel (I)

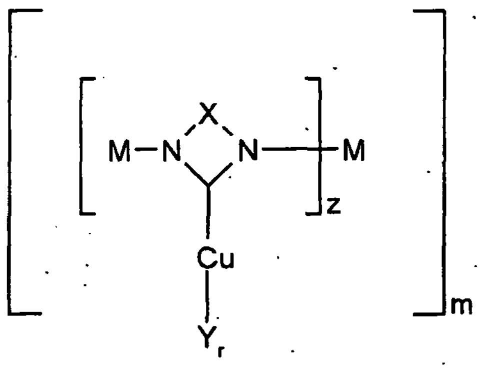

(I)

umgesetzt werden, wobei

X 1,2-Ethandiyl- oder 1,2-Ethendiyl-Rest ist und
M gleich oder verschieden sein kann und für $C_1$-$C_{19}$-Alkyl, $C_7$-$C_{19}$- Aralkyl, $C_6$-$C_{18}$Arylgruppen oder für $C_6$-$C_{19}$-Heteroaryle mit 1 bis 3 Stickstoffatomen steht, wobei zwei oder mehrere Reste M durch ei- ne kovalente Brücke oder durch eine 1 bis 4 Kohlenstoffatome ent- haltene Alkyliden-Brücke oder über einen Aryl- oder Heteroarylring beliebig verbunden sein können,
Y für Halogen, Trifluoracetyl-, Trifluormethansulfonyl-, Nonafluoro- butansulfonyl-, Cyanid-, Acetyl-, fluoriertes Acetylacetonyl-, Nitrat-, Arylsulfonyl-, Oxinat-, Phosphat-, Carbonat- oder Tetrafluorborat- Rest steht,
z für 1, 2 oder 3 steht,
m für ganze Zahlen von 1 bis 6 steht und
r 0, 1 oder 2 bedeutet.

2. Verfahren nach Anspruch 1, wobei die Base ausgewählt ist aus der Gruppe bestehend aus Hydrogencarbonate, Carbonate, Methanolate, Ethanolate, Isopropylate, tert.-Butanolate, Phosphate, Fluoride, Silazane, Hydride und Acetate von Lithium, Natrium, Kalium und Cäsium.

3. Verfahren nach einem der Ansprüche 1 bis 2, wobei der Katalysator der Formel (I) in situ hergestellt wird.

4. Verfahren zur Herstellung von Aminodiphenylamin, wobei das Nitrodiphenylamin nach Anspruch 1 ohne Isolierung hydriert wird.

**Claims**

1. Process for preparing nitrodiphenylamines, wherein nitrohalobenzenes are reacted with anilines, a base and a

catalyst of the formula (I)

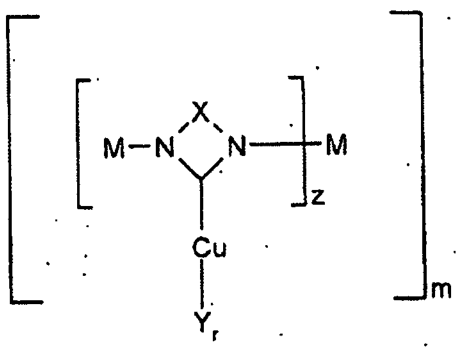

(I)

where

X is a 1,2-ethanediyl or 1,2-ethenediyl radical and
M can be identical or different and are each $C_1$-$C_{19}$-alkyl, $C_7$-$C_{19}$-aralkyl, $C_6$-$C_{18}$-aryl groups or $C_6$-$C_{19}$-heteroaryls having from 1 to 3 nitrogen atoms, where two or more radicals M can be joined in any way by a covalent bridge or by an alkylidene bridge having from 1 to 4 carbon atoms or via an aryl or heteroaryl ring,
Y is halogen or a trifluoroacetyl, trifluoromethanesulphonyl, nonafluorobutanesulphonyl, cyanide, acetyl, fluorinated acetylacetonyl, nitrate, arylsulphonyl, oxinate, phosphate, carbonate or tetrafluoroborate radical,
z is 1,2 or 3,
m is an integer from 1 to 6 and
r is 0, 1 or 2.

2. Process according to Claim 1, wherein the base is selected from the group consisting of hydrogencarbonates, carbonates, methoxides, ethoxides, isopropoxides, tert-butoxides, phosphates, fluorides, silazanes, hydrides and acetates of lithium, sodium, potassium and caesium.

3. Process according to either Claim 1 or 2, wherein the catalyst of the formula (I) is prepared in situ.

4. Process for preparing aminodiphenylamine, wherein the nitrodiphenylamine according to Claim 1 is hydrogenated without being isolated.

**Revendications**

1. Procédé pour la préparation de nitrodiphénylamines, dans lequel on fait réagir des nitrohalogénobenzènes avec des anilines, une base et un catalyseur de formule (I)

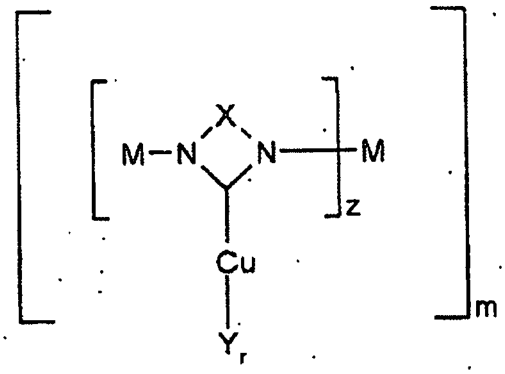

(I)

dans laquelle

X est un groupe 1,2-éthanediyle ou 1,2-éthènediyle et
M peut être le même ou différent et représente des groupes alkyle en $C_1$-$C_{19}$, aralkyle en $C_7$-$C_{19}$, aryle en $C_6$-$C_{18}$ ou des groupes hétéroaryle en $C_6$-$C_{19}$ comportant de 1 à 3 atomes d'azote, deux ou plus de deux radicaux M pouvant être liés au choix par un pont covalent ou par un pont alkylène contenant 1 à 4 atomes de carbone ou par un cycle aryle ou hétéroaryle,
Y représente un atome d'halogène, un radical trifluoroacétyle, trifluorométhanesulfonyle, nonafluorobutanesulfonyle, cyanure, acétyle, acétylacétonyle fluoré, nitrate, arylsulfonyle, oxinate, phosphate, carbonate ou tétrafluoro- borate,
z représente 1, 2 ou 3,
m représente des nombres entiers valant de 1 à 6 et
r représente 0, 1 ou 2.

2. Procédé selon la revendication 1, **caractérisé en ce que** la base est, choisie dans le groupe constitué par les hydrogénocarbonates, carbonates, méthanolates, éthanolates, isopropylates, tert-butanolates, phosphates, fluorures, silazanes, hydrures et acétates de lithium, sodium, potassium et césium.

3. Procédé selon l'une quelconque des revendications 1 et 2, dans lequel le catalyseur de formule (I) est préparé in situ.

4. Procédé pour la préparation d'aminodiphénylamine, dans lequel on soumet la nitrodiphénylamine selon la revendication 1 à une hydrogénation sans isolement.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0166248 A2
- DE 3246151 A
- DE 3501698 A
- DE 185663 A
- US 4670595 A
- US 4187249 A
- US 4683332 A
- US 4187248 A
- US 5840982 A
- US 4404400 A
- JP 63072658 A
- DE 2633811 A


### In der Beschreibung aufgeführte Nicht-Patentliteratur


- **Kirk-Othmer.** Encyclopedia of Chemical Technology. 1992, vol. 3, 424-456
- Ullmann's Encyclopedia of Industrial Chemistry. 1985, vol. A3, 91-111
- Angew. Chem. 1998, vol. 37, 2046-2067
- *Top. Curr. Chem.,* 2002, vol. 219, 131-209
- **Venkatamaran.** *Tetrahedron Letters,* 2001, vol. 42, 4791-4793
- **Buchwald.** *Organic Letters,* 2002, vol. 4, 581-584
- Reductions in Organic Chemistry. ACS Monograph, 188